(19) Europäisches Patentamt
European Patent Office
Office européen des brevets

(11) EP 1 414 876 B1

(12) **EUROPÄISCHE PATENTSCHRIFT**

(45) Veröffentlichungstag und Bekanntmachung des Hinweises auf die Patenterteilung:
**25.03.2009 Patentblatt 2009/13**

(21) Anmeldenummer: **02764649.6**

(22) Anmeldetag: **09.07.2002**

(51) Int Cl.:
***C08F 222/00*** *(2006.01)*

(86) Internationale Anmeldenummer:
**PCT/EP2002/007604**

(87) Internationale Veröffentlichungsnummer:
**WO 2003/014173 (20.02.2003 Gazette 2003/08)**

(54) **DEPOT-POLYMERISATIONSSTARTER-PERLEN**

DEPOT POLYMERIZATION STARTER BEADS

PERLES INITIATRICES DE POLYMERISATION A EFFET RETARD

(84) Benannte Vertragsstaaten:
**AT BE BG CH CY CZ DE DK EE ES FI FR GB GR IE IT LI LU MC NL PT SE SK TR**

(30) Priorität: **08.08.2001 DE 10137968**

(43) Veröffentlichungstag der Anmeldung:
**06.05.2004 Patentblatt 2004/19**

(73) Patentinhaber: **Evonik Röhm GmbH**
**64293 Darmstadt (DE)**

(72) Erfinder:
- **QUIS, Peter**
  **64289 Darmstadt (DE)**
- **HEEB, Heike**
  **64404 Bickenbach (DE)**
- **SCHWIND, Helmut**
  **63457 Hanau (DE)**
- **DRAEGER, Harald**
  **63517 Rodenbach (DE)**

(56) Entgegenhaltungen:
**EP-A- 1 022 295**
**WO-A-01/30872**
**DE-A- 2 355 813**

EP 1 414 876 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Gebiet der Erfindung

Die Erfindung betrifft Suspensionscopolymerisate aus Methylmethacrylat und weiteren, damit copolymerisierbaren Monomeren, die einen bisher nicht erreichten hohen Gehalt an Restperoxiden aufweisen. Die erfindungsgemäßen Perlen mit erhöhtem Restperoxidgehalt können beispielsweise in der Bauchemie als Polymerisationsinitiator in oder redoxhärtenden Bindemitteln angewandt werden.

Stand der Technik

EP 581 387 (Bristol-Myers Squibb Company) beschreibt einen Knochenzement, der aus Polymerpartikeln aufgebaut ist, wobei die Polymerpartikel aus mehreren Schichten bestehen, die unterschiedlichste Additive enthalten können. Neben Röntgenkontrastmitteln, Farbstoffen, Antibiotika, Knochenwachstumsfaktoren können die Schichten auch Polymerisationsinitiatoren enthalten. Die Polymerisatteilchen sind immer schichtförmig aufgebaut. Durch Verteilung des Polymerisationsinitiators auf verschiedene, schalenförmig aufgebaute Compartimente (siehe Figur 7 der EP 581 387) erreicht man unterschiedliche Polymerisationskinetiken.

Aufgabe

Es bestand also die Aufgabe, Polymerisat-Perlen mit unterschiedlicher Zusammensetzung der Ausgangs-Monomeren und möglichst hohen Gehalten an homogen verteiltem Polymerisationsstarter zur Verfügung zu stellen, die ohne den komplizierten schalenförmigen Aufbau auskommen und so einfacher und preiswerter herzustellen sind. Die Perlen sollen als Polymerisations-Initiator weiter über einen großen Verarbeitungsspielraum in monomerhaltigen radikalisch härtbaren Reaktionsharz-Systemen verfügen, so dass die Topfzeit möglichst groß sein soll.

Gelöst wurde die Aufgabe durch eine Depot-Polymerisationsstarter Perle gemäß den Ansprüchen.

Die erfindungsgemäße Perle verfügt über folgende Vorteile:

- Über das Verhältnis von Peroxidkonzentration zu Polymer und über die Löslichkeit der Perlen im Reaktionsharz lässt sich die offene Zeit, die zur Verarbeitung des Reaktionsharzes zur Verfügung stellt, gut steuern.

- Durch einen gleichmäßigen Radikalstrom, sowohl räumlich als auch zeitlich gesehen, erhält man eine bessere Durchhärtung des Reaktionsharzes im Vergleich zu den üblichen Polymerisationsstartern.

- Man erreicht auch eine bessere Oberflächentrockenheit der formulierten Beschichtungssysteme vor Ort.

Zusammensetzung der erfindungsgemäßen Perlen:

1. 1 - 99 Gew.-% eines Derivats einer ungesättigten Carbonsäure der Formel (I),

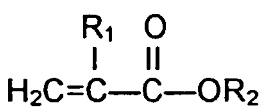

(I)

wobei gilt:

$R_1$ = H oder $CH_3$
$R_2$ = Methyl, Ethyl

2. 99- 1 Gew.-% eines Derivats einer ungesättigten Carbonsäure der Formel (II),

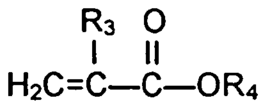

(II)

wobei gilt:

$R_3$=HoderCH$_3$
$R_4$ = ein aliphatischer Rest mit 4-6 Kohlenstoffatomen, wie beispielsweise:

n-Butyl, iso-Butyl, t-Butyl, Pentyl, Hexyl und MethylCyclopentyl, Cyclohexyl, ferner Terahydrofurfuryl oder Isobornyl

3. 1 -10 Gew.-% eines Peroxids oder eines Peroxidgemischs.
Bevorzugt werden 3-10 Gew.-% eines Peroxids oder eines Peroxidgemischs. Besonders bevorzugt werden 5-10 Gew.-% eines Peroxids oder eines Peroxidgemischs
Als Peroxid oder Peroxidgemisch werden beispielsweise Benzoylperoxide und andere Diacylperoxide eingesetzt.

4. 1 - 15 Gew.-% eines oder mehrer mit den Monomeren unter 1. oder 2. copolymerisierbaren Monomeren, wobei sich die Prozentsätze von 1. - 4. zu 100 % addieren.

Als copolymerisierbare Monomere werden beispielsweise Styrol oder Styrolderivate, wie beispielsweise Methylstyrol oder Maleinsäure oder Maleinsäureanhydrid eingesetzt.
Die Perlen haben einen mittleren Durchmesser von 50 bis 200 µm. Der Durchmesser wurde mittels Laserbeugungsspektroskopie mit einem Mastersizer Microplus der Fa. Malvern (Messbereich: 0,05 - 555 µm) bestimmt.
Der Restmonomergehalt wurde mittels der gaschromatographischen Headspace-Analyse, einer Methode zur Bestimmung verdampfbarer Bestandteile in Flüssigkeiten und Feststoffen (u. a. von Monomeren in Thermoplasten) gemessen.
Die Viskositätszahl VZ (auch Staudinger Funktion) ist die konzentrationsbezogene relative Viskositätsänderung einer 0,5%igen Lösung des Copolymeren in Chloroform bezogen auf das Lösungsmttel, wobei die Durchlaufzeiten im Ubbelohde-Viskosimeter mit hängendem Kugelniveau, Schott Typ Nr. 53203 und Kapillare 0c nach DIN-Norm 51562 bei 25°C ermittelt wurden. Als Lösungsmittel diente Chloroform.

$$VZ = \left( \frac{t}{t_o} - 1 \right) . \frac{1}{c}$$

wobei:

t = Durchlaufzeit der Polymerlösung in Sekunden
$t_o$ = Durchlaufzeit des Lösemittels in Sekunden
c = Konzentration in g/100 ccm

Herstellungsvorschrift

Die Herstellung der erfindungsgemäß zu verwendenden Copolymere ist an sich bekannt. Sie können in Substanz- oder Suspensionspolymerisation hergestellt werden. Hilfreiche Hinweise finden sich hinsichtlich der Substanzpolymerisation bei Houben-Weyl, Band E20, Teil 2 (1987), Seite 1145 ff. Die Suspensionspolymerisationstechnik wird eben dort auf Seite 1149 ff beschrieben.

BEISPIELE

Beispiel 1

Herstellvorschrift der Depotperoxid Perle:

In einem 5l Polymerisationsgefäß, ausgestattet mit Rührer, Rückflusskühler und Thermometer, wurden 37,75 g Aluminiumsulfat x 14 $H_2O$ in 3750 g vollentsalztem Wasser gelöst und auf 40 °C erwärmt. Zur Erzeugung des Suspensionsstabilisators wurden 166,25 g einer 10%igen wässrigen Natriumcarbonat-Lösung, 0,28 g Natrium-$C_{15}$-Paraffinsulfonat und 0,272 g Polyethylenglykol (Molgewicht 5 000 - 6 000) unter Rühren zugegeben. Unter weiterem Rühren wurden nun 1250 g eines Gemisches aus 43,1 Gewichtsteilen Methylmethacrylat, 43,1 Gewichtsteilen n-Butylmethacrylat, 0,013 Gewichtsteilen Thioglykolsäure-2-ethylhexylester und 13,7 Gewichtsteilen 75%igem, wässrigen Dibenzoylperoxid zugegeben. Der Ansatz wurde anschließend auf 70°C erwärmt, 40 Minuten bei 70°C und 60 Minuten bei 77°C polymerisiert und dann auf 50°C abgekühlt. Bei dieser Temperatur wurden zur Auflösung des Suspensionsstabilisators 36 g 50%ige Schwefelsäure zugesetzt. Nach weiterem Abkühlen auf Raumtemperatur wurden die Polymerisationsperlen abfiltriert, gründlich mit vollentsalztem Wasser ausgewaschen und im Wirbelbett-Trockner bei 40 °C getrocknet.
Es wurden 1 391 g klare Polymerisatperlen mit einem jodometrisch ermittelten Rest-Dibenzoylperoxid-Gehalt von 10 Gew.-% erhalten.

Beispiel 2

Aushärtversuch mit einem Reaktionsharz auf Basis hochsiedender (Meth)acrylsäureester (HS):
Die unter Beispiel 1 beschriebene Peroxidperle mit einem Gehalt von 10 % Dibenzoylperoxid wurde als Reaktionsstarter in einem Reaktionsharz auf Basis hochsiedender (Meth)acrylester in einer Konzentration von 10 % (= 1 % aktives Dibenzoylperoxid) unter Rühren innerhalb von 2 Minuten eingebracht. Das aktivierte Reaktionsharz wurde dann in einer Schichtdicke von ca. 2 mm auf eine Betonunterlage aufgegossen. Es härtete innerhalb von ca. 30 Minuten klebfrei aus und wies eine Topfzeit von 15 bis 18 Minuten auf.

Beispiel 3

Vergleichsversuch mit phlegmatisiertem Dibenzoyl-Peroxid:
Der Versuch von Beispiel 2 wurde wiederholt, als Starter jedoch 2 % Dibenzoylperoxid 50 %ig in Dicyclohexylphthalat phlegmatisiert (BP-50-FT, Interox) eingesetzt. Das Reaktionsharz härtete ebenfalls innerhalb von ca. 30 Minuten aus. Die Topfzeit betrug jedoch nur ca. 8 - 10 Minuten.

**Topfzeiten/Verarbeitungszeiten von Bindemitteln - Basis: MMA, nBuMA oder Hochsieder mit verschiedenen Depot-Peroxid-Perlen**

| Harz /<br>TZ+HZ*<br>*Topfzeit/Härtezeit | BPO-50% -Pulver- | **Depot-Perle** (10% Peroxid-Gehalt)<br>MMA | MMA+10ppmBDMA | MMA/ BuMA=50/50 | MMA/ BuMA=75/25 | MMA/ BuMA=25/75 |
|---|---|---|---|---|---|---|
| Gew. % | 2 | 10 | 10 | 10 | 10 | 10 |
| DGD 523<br>"MMA" | 8-10' / 18-23'<br>116°C-17'<br>l. gelbl. | schl. Lösl.<br>~50 / 75-100'<br>**klar, l.feucht** | schl. Lösl.<br>~55 / 85-110'<br>Randber.feucht<br>Oberfl. Rauh | gelartig<br>6' / 15-20'<br>134°C-15'<br>Gelknubbel | 6-8' / 18-22'<br>119°C-18'<br>s.rauhe Oberfl. | unlöslich |
| | | | | | | schl. Lösl.-gelart. |

(fortgesetzt)

| Topfzeiten/Verarbeitungszeiten von Bindemitteln - Basis: MMA, nBuMA oder Hochsieder mit verschiedenen Depot-Peroxid-Perlen | | | | | | |
|---|---|---|---|---|---|---|
| | | | | | | |
| Harz / | BPO-50% -Pulver- | **Depot-Perle** (10% Peroxid-Gehalt) | | | | |
| TZ+HZ* *Topfzeit/Härtezeit | | MMA | MMA+10ppmBDMA | MMA/ BuMA=50/50 | MMA/ BuMA=75/25 | MMA/ BuMA=25/75 |
| NGB 52 "BuMA" | 8-10' / 30-40' 90°C-19' l. gelbl. | > 5 h Abbruch | > 5 h Abbruch | 15' / 35' gelblich | 70' /120-170' 90°C-90' **klar** | 7-9' /20-30' Oberfl. Rauh |
| GFG 330 HS | 4-6' / 15-20' l. gelbl. | > 5 h Abbruch | > 5 h Abbruch | 10-12' / 22-30' 104°C-17' **klar** | 45' ungleichm. keine Härtg. | gelartig 5-6' / 13-18' 96°C-11' |

Degadur 523 (DGD 523) wird von der Röhm GmbH & Co. KG in den Handel gebracht und stellt ein Reaktionsharz dar, das auf Basis von niedrigsiedenden Monomeren hergestellt wird. Die Monomerphase von Degadur 523 besteht aus Methylmethacrylat, 2-Ethyl-hexylacrylat und mehrfunktionellen Methacrylaten. NGB52 ist ein Reaktionsharz auf der Basis von mittelsiedeneden Monomeren (z. B. Butylmethacrylat). GFG 330 HS ist ein Reaktionsharz auf der Basis von hochsiedenen Monomeren, wie z. B. Tetrahydrofurturylmethacrylat.

An der ersten Stelle steht in der Tabelle die Topfzeit, an zweiter Stelle die Aushärtezeit. Die Topfzeit sollte zwischen 50 und 20 Minuten betragen, die Aushärtezeit zwischen 25 und 60 Minuten. Die Beschichtungsmasse ist ein homogenes Gemisch, das sich gut auftragen lässt.

## Patentansprüche

**1.** Suspensionscapolymerisat, zusammengesetzt aus folgenden Komponenten:

1. Mehr als 1 Gew.-% eines Derivats einer ungesättigten Carbonsäure der Formel (I),

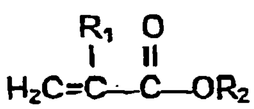

(I)

wobei gilt:

$R_1$ = H oder $CH_3$
$R_2$ = Methyl, Ethyl

2. Mehr als 1 Ges.-% eines Derivats einer ungesättigten Carbonsäure der Formel (II),

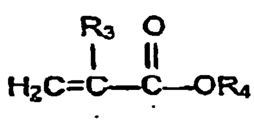

(II)

wobei gilt:

$R_3$ = H oder $CH_3$
$R_4$ = n-Butyl, iso-Butyl

3. 1 -10 Gew.-% eines Peroxids oder eines Peroxidgemischs
4. 1 - 15 Gew.-% eines oder mehrer mit den Monomeren unter 1. oder 2. copolymerisierbaren Monomeren, wobei sich die Prozentsätze von 1. - 4. zu 100 % addieren.

**2.** Verwendung der Perlen nach Anspruch 1 als Poylmerisationsstarter in Bodenbeschichtungsmittel und Reaktionsharzbasis.

**3.** Verwendung der Perlen nach Anspruch 1 zur Herstellung von Dentalfüllmassen.

**4.** Verwendung der Perlen nach Anspruch 1 zur Herstellung von Kaltplastikstraßenmarkierungsmassen.

## Claims

**1.** Suspension copolymer composed of the following components:

1. more than 1% by weight of a derivative of an unsaturated carboxylic acid of the formula (I),

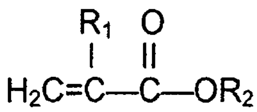

(I)

where:

$R_1$ = H or $CH_3$
$R_2$ = methyl, ethyl

2. more than 1% by weight of a derivative of an unsaturated carboxylic acid of the formula (II),

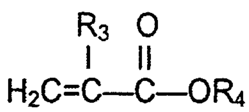

(II)

where:

$R_3$ = H or $CH_3$
$R_4$ = n-butyl, isobutyl

3. from 1-10% by weight of a peroxide or of a peroxide mixture
4. from 1-15% by weight of one or more monomers copolymerizable with the monomers of 1. or of 2., where the percentages of 1.-4. give a total of 100%.

**2.** Use of the beads according to claim 1 as polymerization initiators in a floorcovering composition and reactive resin basis.

**3.** Use of the beads according to claim 1 for producing dental filling compositions.

**4.** Use of the beads according to claim 1 for producing cold-plastic road-marking compositions.

**Revendications**

**1.** Produit de copolymérisation en suspension, composé des composants suivants :

1. plus de 1 % en poids d'un dérivé d'un acide carboxylique insaturé de formule (I)

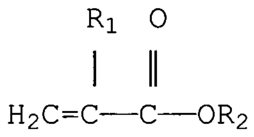

(I)

dans laquelle

$R_1$ = H ou $CH_3$
$R_2$ = méthyle, éthyle,

2. plus de 1 % en poids d'un dérivé d'un acide carboxylique insaturé de formule (II)

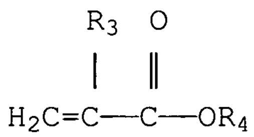

(II)

dans laquelle

$R_3$ = H ou $CH_3$
$R_4$ = n-butyle, isobutyle,

3. 1-10 % en poids d'un peroxyde ou d'un mélange de peroxydes,
4. 1-15 % en poids d'un ou plusieurs monomère(s) copolymérisable(s) avec les monomères en 1. ou 2.,

la somme des pourcentages de 1-4 étant égale à 100 %.

**2.** Utilisations des perles selon la revendication 1, en tant qu'amorceur de polymérisation dans des compositions de revêtement de sols et des bases de résines réactives.

**3.** Utilisation des perles selon la revendication 1, pour la préparation de matières de plombage dentaire.

**4.** Utilisation des perles selon la revendication 1, pour la préparation de masses pour marquage routier plastique à froid.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 581387 A